# EUROPÄISCHE PATENTANMELDUNG

(11) **EP 1 529 549 A1**
(43) Veröffentlichungstag der Anmeldung: **11.05.2005**
(21) Anmeldenummer: 04025934.3
(22) Anmeldetag: 02.11.2004
(51) Int. Cl.: A61M 39/20, B65D 41/04, B65D 39/08, B65D 51/22, B65D 39/16, A61M 39/10

(54) **Vorrichtung zum Verbinden, Abdecken oder Verschliessen von Schläuchen, Behältern oder dergleichen Gegenstände**

(30) Priorität: 04.11.2003 DE 20316930 U
(71) Anmelder: B. BRAUN MELSUNGEN AG, 34212 Melsungen (DE)
(72) Erfinder: Held, Fred, 22299 Hamburg (DE)
(74) Vertreter: Müller, Hans-Jürgen, Dipl.-Ing.

(57) **Zusammenfassung**

Bei einer Vorrichtung zum Verbinden, Abdecken und/oder Verschließen von Schläuchen, Behältern oder dergl. Gegenständen insb. des medizinischen Bedarfs sind zur besseren Trennbarkeit unterschiedlicher Funktionen der Funktionsteil (3, 3a), der zum Ein- oder Aufsetzen in bzw. auf einen zugehörigen Gegenstand dient, und der Bedienteil (1, 1a) zum Handhaben des Funktionsteils durch einen Trennteil (4) getrennt, welcher in mindestens zwei Richtungen über den Bedienteil und/oder den Funktionsteil hinausragt.

## Beschreibung

Die Erfindung bezieht sich auf eine Vorrichtung zum Verbinden und/oder Abdecken bzw. Verschließen von Schläuchen, Behältern oder dergleichen Gegenständen der im Oberbegriff des Anspruchs 1 genannten Gattung. Derartige Vorrichtungen werden insbesondere in der Medizintechnick gebraucht, um beispielsweise als Luer-Lock-Verbindung zwischen Schlauchsystemen oder zwischen Schläuchen und Aufnahmebehältern und medizinischen Instrumenten und Geräten zu dienen. Dabei ist es wichtig, dass das Bedienungspersonal solcher Vorrichtungen genügend Sorgfalt walten lässt, damit Funktionsteile möglichst frei bleiben von Verunreinigungen, wie sie beispielsweise beim Handhaben der Vorrichtung dort hinterlassen werden können.

Der Erfindung liegt daher die Aufgabe zugrunde, eine derartige Vorrichtung auf einfache Weise dahingehend zu verbessern, dass die Teile unterschiedlicher Funktion - zum Ein- oder Aufsetzen der Vorrichtung in bzw. auf einen zugehörigen Gegenstand einerseits und zum Handhaben des erstgenannten Teil beim Bedienen andererseits - gut voneinander trennbar sind.

Die Erfindung ist in den Ansprüchen 1, 6 und 21 gekennzeichnet und in Unteransprüchen sowie in der Figurenbeschreibung sind besondere Ausführungsbeispiele beansprucht und im Detail beschrieben.

Die Aufgabe der mechanischen Trennung erfolgt gemäß der Erfindung dadurch, dass der Bedienteil vom Funktionsteil durch einen Trennteil getrennt ist, welcher in mindestens zwei Richtungen über den Bedienteil und/oder den Funktionsteil hinausragt. Dies geschieht insbesondere dadurch, dass der Trennteil als ringförmiger Flansch ausgebildet ist und radial mindestens über den Bedienteil hinausragt. Beim Erfassen des Bedienteils wird hierdurch vermieden, dass die Finger der Bedienungsperson auch den Funktionsteil erfassen; der Trennteil verhindert das "Übergreifen" vom Bedienteil auf den Funktionsteil.

Die Aufgabe einer auch optisch guten Trennung der beiden Teile wird beispielsweise dadurch gelöst, dass der Bedienteil eine optisch vom Funktionsteil deutlich abweichende Farbmarkierung beispielsweise in Form eines farbigen Ringes oder Streifens aufweist. Die Aufmerksamkeit der Bedienungsperson wird dann zwangsläufig auf diesen Bedienteil gelenkt, um die Vorrichtung dort zu erfassen. Diese Aufgabe der optischen Trennung wird auch dadurch gelöst, dass nach einer weiteren Ausbildung der Erfindung der Bedienteil Rippen bzw. Rillen oder andere Erhöhungen aufweist. Diese oder auch eine an der Oberfläche angebrachte Feinstruktur verhindern darüber hinaus das Abrutschen der Finger; sie sichern einen festen Halt beim Ergreifen der Vorrichtung.

Der Funktionsteil ist insbesondere rohrförmig, bevorzugt zylinderförmig ausgebildet.

Der Bedienteil sollte hülsenförmig ausgebildet sein.

Es empfiehlt sich, wenn der Bedienteil axial nach der einen Seite und der Funktionsteil radial nach der anderen Seite vom Trennteil abstehen.

Der Bedienteil sollte im Querschnitt aussen rechteckförmig insbesondere mit abgerundeten Ecken des aussen rechteckförmigen Querschnitts ausgebildet sein. Dies erleichtert das Ergreifen zweier länglicher paralleler Oberflächen, die zweckmäßigerweise quer zur Axialrichtung der Vorrichtung mit den Rippen bzw. Rillen versehen sind. Dies ist gleichzeitig eine optische Signalisierung dafür, dass eine solche Vorrichtung in Axialrichtung in Bezug zum oder vom zugehörigen Gegenstand auf diesen aufgesetzt, in diesen eingesetzt oder von diesem abgezogen werden soll.

Werden dagegen bevorzugt rotierende oder Drehbewegungen der Vorrichtung in Bezug zu dem anderen zugehörigen Gegenstand, mit dem eine Verbindung hergestellt oder gelöst werden soll, angestrebt, dann empfiehlt sich, die Rippen bzw. Rillen in Axialrichtung verlaufen zu lassen; in diesem Fall wird ein Bedienteil mit einem im Querschnitt zylinderförmigen Umfang bevorzugt.

Sofern der Funktionsteil bzw. der Bedienteil stirnseitig verschlossen sind, eignet sich die Vorrichtung als Abdeckung.

Sofern Bedienteil und Funktionsteil einen axialen Durchlass aufweisen, stellt die Vorrichung ein Verbindungselement zwischen anderen Gegenständen dar, um beispielsweise Flüssigkeit oder andere Medien durch die Vorrichtung vom einen zum anderen Gegenstand hindurchzulassen.

Falls die Vorrichtung als Verbindungsteil zum Durchlassen von Medien von einem Gegenstand zu einem anderen Gegenstand verwendet werden soll, empfiehlt sich die Verwendung eines transparenten oder semitransparenten Kunststoffs, um das betreffende Medium optisch sichtbar zu machen.

Für manche Anwendungsfälle empfiehlt sich, wenn sich an den Bedienteil eine Aufnahmehülse axial anschließt; diese kann zur Aufnahme von solchen Gegenständen dienen, die nach aussen geschützt werden sollen.

Die Enden des Bedienteils sollten abgerundet sein, um einer Verletzungsgefahr zu begegnen. Im übrigen empfiehlt sich eine nach ergometrischen und haptischen Gesichtspunkten ausgebildete Formgestalt des Bedienteils.

Wird die Vorrichtung als Abdeckung für Schläuche, Behälter oder andere entsprechende Vorrichtungen verwendet, empfiehlt sich die Verwendung eines nicht-transparenten Kunststoffs ohne einen Durchlass; hierdurch wird optisch signalisiert, dass sich diese Vorrichung nicht als Verbindungselement, sondern als "Abdeckkappe" eignet und bei Anschluss eines anderen Verbindungsteils oder Gegenstands zuerst abgenommen werden muß.

Im Falle einer solchen "Abdeckkappe" kann es sich empfehlen, dass der Funktionsteil und/oder der Bedienteil aus einem Kunststoff oder anderem Material besteht, der durch einen spitzen Gegenstand, wie eine Hohlnadel oder einen Spike, durchstoßbar bzw. durchbrechbar ist.

Ferner empfiehlt es sich, wenn der Funktionsteil ein Rastelement aufweist, das bei Herstellung bzw. Vervollständigung der Verbindung zu dem zugehörigen Gegenstand an diesem oder in diesen einrastet. Ratschenartige, clickartige und dergleichen Rastelemente sind bereits bekannt; sie signalisieren auch akustisch die Herstellung der Verbindung.

Bevorzugte Ausbildungen der Erfindung werden im folgenden anhand der Zeichnungen näher erläutert. Dabei zeigen
- Figur 1: eine erfindungsgemäße Vorrichtung, die aus zwei Teilen besteht, von denen der eine Teil am Ende eines Schlauches und der andere Teil am Ende eines anderen Schlauches angebracht sind. Dabei ist Figur 1a eine Darstellung in Seitenansicht und Figur 1b eine Darstellung in Aufsicht auf die Luer-Lock-Verbindung der beiden Vorrichtungsteile;
- Figur 2a: drei Ansichten einer Ausbildung der erfindungsgemäßen Vorrichtung und
- Figur 2b: drei Ansichten einer anderen Ausbildung einer erfindungsgemäßen Vorrichtung;
- Figur 3: eine Ausbildung der Erfindung als Verschlußkappe auf einem Behälter in Seitenansicht;
- Figur 4: eine Seitenansicht auf eine weitere Ausbildung der Erfindung, bei der sich an den Bedienteil eine Abdeckhülse zum Abdecken einer Nadel oder eines Spikes anschließt und
- Figuren 5 und 6: ein als Öffnungsvorrichtung für einen verschlossenen Behälter dienendes und an einem Ende spitzes Spike-Element.

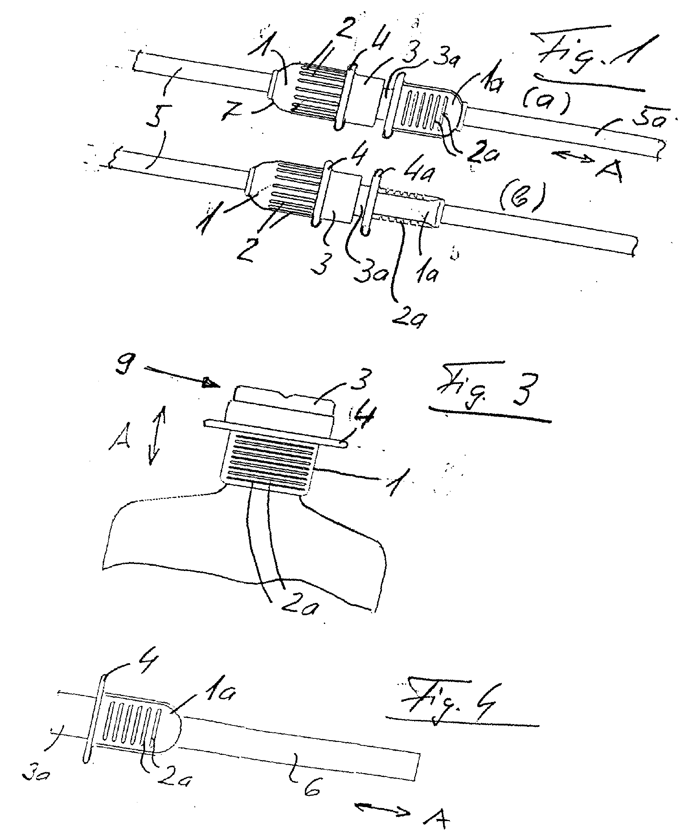

Die in Figur 1 links dargestellte Vorrichtung weist einen aussen zylinderförmigen Bedienteil 1 auf, der mit in Axialrichtung verlaufenden Rillen 2 versehen ist, die über den Umfang des Bedienteils 1 verteilt sind. Dieser ist vom aussen zylindrischen Funktionsteil 3 durch einen Trennteil 4 getrennt, welcher als ringförmiger Flansch einen größeren Durchmesser als der Bedienteil 1 und der Funktionsteil 2 aufweist. Der Funktionsteil 3 dient zum Verbinden mit dem Funktionsteil 3a der jeweils links dargestellten Vorrichtung, bei der der Bedienteil 1a vom Funktionsteil 3a gleichfalls durch einen als ringförmigen Flansch ausgebildeten Trennteil 4a getrennt ist. Im Unterschied zu der links dargestellten Vorrichtung ist der Bedienteil 1a der rechts dargestellten Vorrichtung dagegen im Aussenquerschnitt im wesentlichen rechteckig mit abgerundeten Kanten geformt. Dabei sind die Rippen 2a bzw. Rillen quer zur Axialrichtung angeordnet. Die Enden 7 des Bedienteils 1 sind abgerundet.

Bei dieser Luer-Lock-Verbindung der beiden Vorrichtungsteile wird der Bedienteil 1a der rechts dargestellten Vorrichtung in Bezug zum links dargestellten Vorrichtungsteil in Axialrichtung A bewegt, während der links dargestellte Vorrichtungsteil in rotierende d.h. in drehende Bewegung gesetzt werden kann, um die Verbindung herzustellen oder wieder zu trennen. Der Funktionsteil 3a paßt (Pass-Sitz) satt und dicht stöpselartig in den Funktionsteil 3.

Beide Vorrichtungsteile bestehen aus semitransparentem grünlichem Kunststoff, so dass auch von aussen sichtbar ist, wenn ein Medium, beispielsweise Flüssigkeit, von einem Schlauch 5 durch die aus den beiden Vorrichtungen bestehende Verbindung in den anderen Schlauch 5a fließt.

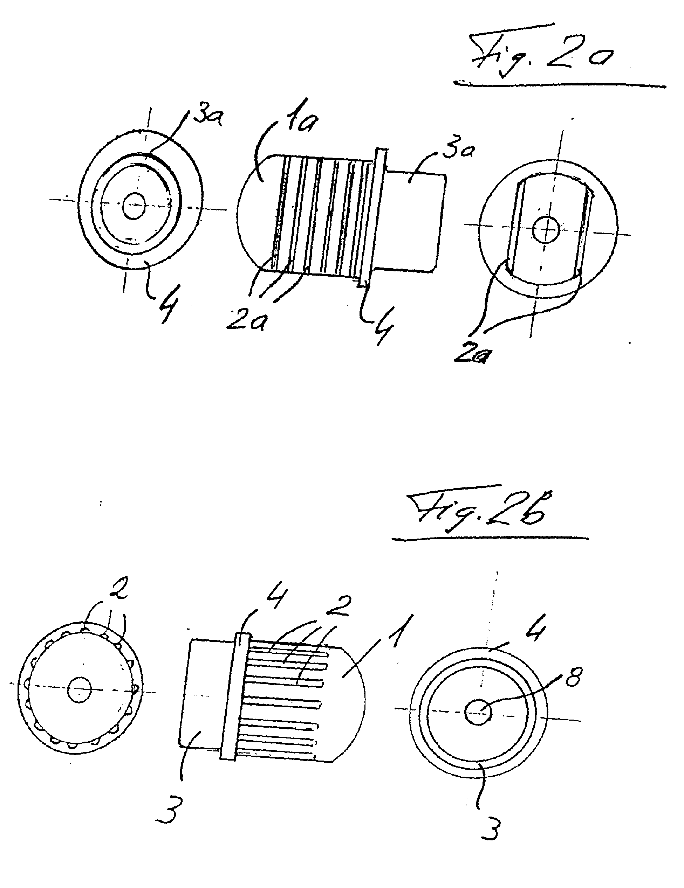

Die erfindungsgemäße Formgebung wird noch deutlicher in Figur 2 gezeigt. Die Rippen 2, 2a stehen dabei um 1,6 mm mit einem Radius von 0,25 mm von der Oberfläche des Bedienteils 1, 1a ab. Zwischen den Rippen 2, 2a kann eine zusätzliche Feinriffelung als Feinstruktur mit 0,25 mm Höhe über der Oberfläche und einem Kantenradius von 0,05 mm angeordnet sein.

Im Falle von konzentrischen Ringen auf dem Bedienteil 1 empfiehlt sich die Verwendung von etwa 3 bis 5 Ringen bei einem Abstand derselben von etwa 1,6 mm. Die Höhe der ringförmigen Stege sollte wie die der Rippen etwa 1,6 mm betragen.

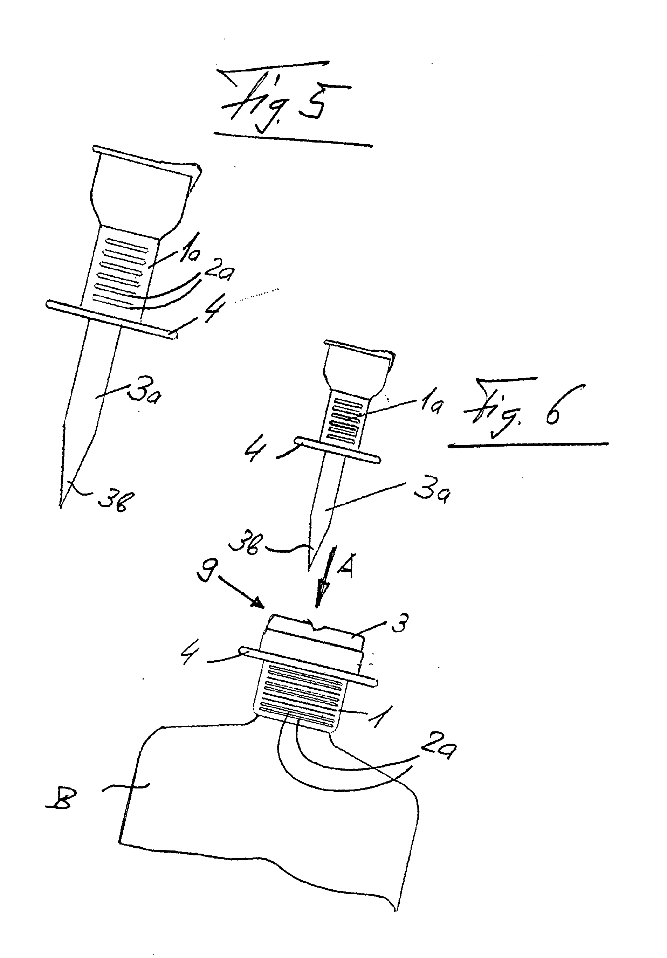

Bei der als Verschlusskappe dienenden Vorrichtung nach Figur 3 dient der Bedienteil 1 zum Erfassen der Vorrichtung. Er ist gleichfalls mit quer zur Axialrichtung der Vorrichtung dienenden Rippen 2a versehen und vom Funktionsteil 3 durch einen als Ringflansch ausgebildeten Trennteil 4 abgetrennt. Der Funktionsteil 3 kann am Stirnende verschlossen sein und als Behälterabdeckung 9 dienen, um erst bei Bedarf, beispielsweise durch Durchstoßen, geöffnet und mit einem anderen Verbindungsteil eines medizinischen Geräts oder Instruments verbunden zu werden.

Gemäß Figur 4 setzt sich der hülsenartige und im Querschnitt wiederum im wesentlichen rechteckförmige Bedienteil 1a in einer rohrförmigen Aufnahmehülse 6 fort und zwar in eine Richtung, die vom Funktionsteil 3a abgewandt ist. Eine derartige Vorrichtung kann als "sterile Kappe" an Spike-/Nadelverbindungen verwendet werden. Die quer zur Axialrichtung A verlaufenden Rippen 2a deuten an, dass diese Vorrichtung vom betreffenden zugeordneten Gegenstand abzuziehen ist. Wäre der Bedienteil dagegen im Querschnitt zylinderförmig mit in Axialrichtung verlaufenden Rippen, so würde dies als Indiz dafür gedeutet, dass die Vorrichtung durch Drehen vom zugehörigen Gegenstand zu trennen ist.

Nach Figur 5 weist ein als "Spike" bzw. spitzes Öffnungselement für insbesondere Behälterabdeckungen 9 dienende Vorrichtung ebenfalls einen als Ringflansch dienenden Trennteil 4 auf, von dem sich in Axialrichtung A einerseits ein Funktionsteil 3a mit einer Spitze 3b und andererseits - in der entgegengesetzten Richtung - ein Bedienteil 1a mit quer zur Axialrichtung A verlaufenden Rippen 2a erstrecken.

Mit der Spitze 3a kann in die Oberfläche der Behälterabdeckung 9 gemäß Figur 5 eingestochen werden. Dadurch wird der vorher aseptisch verschlossene Behälter B, z.B. eine Sterilflasche, geöffnet. Der Trennteil 4 sorgt dafür, dass die Finger der Bedienperson vom Bedienteil 1 nicht bis auf den Funktionsteil 3a übergreifen können. Diese Öffnungsvorrichtung ist bevorzugt aus transparentem und grünem Kunststoff hergestellt.

Die Öffnungsvorrichtung ist bevorzugt hohl, insbesondere als "Hohlnadel" ausgebildet, um durch diese hindurch den Inhalt des Behälters B z.B. mit Zusatzstoffen zu befüllen und/oder zum Gebrauch des Behälterinhalts leicht und ohne Verschmutzung entleeren zu können.

## Patentansprüche

1. Vorrichtung zum Verbinden und/oder Abdecken bzw. Verschließen von Schläuchen, Behältern oder dergleichen Gegenständen, insbesondere des medizinischen Bedarfs mit einem Funktionsteil (3, 3a) zum Ein- oder Aufsetzen in bzw. auf einen zugehörigen Gegenstand und mit einem Bedienteil (1, 1a) zum Handhaben des Funktionsteils (3, 3a),
**dadurch gekennzeichnet,**
**dass** der Bedienteil (1, 1a) vom Funktionsteil (3, 3a) durch einen Trennteil (4) getrennt ist, welcher in mindestens zwei Richtungen über den Bedienteil (1, 1a) und/oder den Funktionsteil (3, 3a) hinausragt.

2. Vorrichtung nach Anspruch 1,
**dadurch gekennzeichnet,**
**dass** der Trennteil (4) als ringförmiger Flansch ausgebildet ist und radial über den Bedienteil (1, 1a) hinausragt.

3. Vorrichtung nach Anspruch 1 oder 2,
**dadurch gekennzeichnet,**
**dass** der Funktionsteil (3, 3a) rohrförmig ausgebildet ist.

4. Vorrichtung nach einem der vorhergehenden Ansprüche,
**dadurch gekennzeichnet,**
**dass** der Bedienteil (1) hülsenförmig ausgebildet ist.

5. Vorrichung nach einem der vorhergehenden Ansprüche,
**dadurch gekennzeichnet,**
**dass** der Bedienteil (1, 1a) axial nach der einen Seite und der Funktionsteil (3, 3a) axial nach der anderen Seite des Trennteils (4) von diesem abstehen.

6. Vorrichtung insbesondere nach einem der vorhergehenden Ansprüche,
**dadurch gekennzeichnet,**
**dass** der Bedienteil (1, 1a) mindestens auf Teilen der Oberfläche mit Rippen (2, 2a) und/oder Rillen versehen ist.

7. Vorrichtung nach Anspruch 6,
**dadurch gekennzeichnet,**
**dass** sich die Rippen (2) bzw. Rillen in Axialrichtung (A) des Bedienteils (1, 1a) erstrecken.

8. Vorrichtung nach Anspruch 6,
**dadurch gekennzeichnet,**
**dass** sich die Rippen (2a) bzw. Rillen in Umfangsrichtung und/oder quer zur Axialrichtung (A) erstrecken.

9. Vorrichtung nach einem der vorhergehenden Ansprüche,
**dadurch gekennzeichnet,**
**dass** der Bedienteil (1) mindestens auf Teilen der Oberfläche mit konzentrischen Kreisen versehen ist.

10. Vorrichtung nach einem der vorhergehenden Ansprüche,
**dadurch gekennzeichnet, dass** die Oberfläche des Bedienteils (1, 1a) mindestens teilweise mit einer das Abgleiten von Fingern erschwerenden Feinstruktur versehen ist.

11. Vorrichtung nach einem der vorhergehenden Ansprüche,
**dadurch gekennzeichnet,**
**dass** sich axial an den Bedienteil (1a) eine Aufnahmehülse (6) anschließt.

12. Vorrichtung nach einem der vorhergehenden Ansprüche,
**dadurch gekennzeichnet,**
**dass** der Funktionsteil (3, 3a) stirnseitig verschlossen ist.

13. Vorrichtung nach einem der vorhergehenden Ansprüche,
**dadurch gekennzeichnet,**
**dass** der Bedienteil (1, 1a) stirnseitig verschlossen ist.

14. Vorrichtung nach einem der vorhergehenden Ansprüche,
**dadurch gekennzeichnet,**
**dass** die Enden (7) des Bedienteils (1, 1a) abgerundet sind.

15. Vorrichtung nach einem der vorhergehenden Ansprüche,
**dadurch gekennzeichnet,**
**dass** der Funktionsteil (3, 3a) zylinderförmig ausgebildet ist.

16. Vorrichtung nach einem der vorhergehenden Ansprüche,
**dadurch gekennzeichnet,**
**dass** der Bedienteil (1a) im Querschnitt aussen rechteckförmig ausgebildet ist.

17. Vorrichtung nach Anspruch 16,
**dadurch gekennzeichnet,**
**dass** die Ecken des aussen rechteckförmigen Querschnitts abgerundet sind.

18. Vorrichtung nach einem der vorhergehenden Ansprüche zur Verwendung als Schlauchverbindung, insbesondere Luer-Lock-Verbindung, **gekennzeichnet durch** einen transparenten oder semitransparenten Kunststoff und **durch** einen axialen Durchlass (8).

19. Vorrichtung nach einem der Ansprüche 1 bis 17 zur Verwendung als Schlauch- oder Behälterabdeckung **gekennzeichnet durch** einen nicht-transparenten Kunststoff ohne einen Durchlass.

20. Vorrichtung nach Anspruch 19,
**dadurch gekennzeichnet,**
**dass** der Funktionsteil (1, 1a) und/oder der Bedienteil (3, 3a) aus einem Kunststoff besteht, der durch einen spitzen Gegenstand durchstoßbar bzw. durchbrechbar ist.

21. Vorrichtung insbesondere nach einem der vorhergehenden Ansprüche,
**dadurch gekennzeichnet,**
**dass** der Bedienteil (1, 1a) eine vom Funktionsteil (3, 3a) optisch deutlich abweichende Farbmarkierung aufweist.

22. Vorrichtung nach einem der vorhergehenden Ansprüche,
**dadurch gekennzeichnet,**
**dass** der Funktionsteil (1, 1a) ein Rastelement aufweist, das bei Herstellung der Verbindung an bzw. im zugehörigen Gegenstand einrastet.

23. Vorrichtung nach einem der vorhergehenden Ansprüche,
**dadurch gekennzeichnet,**
**dass** der Funktionsteil (3a) an einem Ende eine Spitze (3b) zum Einstechen in und/oder Durchstoßen durch eine Behälterabdeckung (9) aufweist.
